# EUROPÄISCHE PATENTSCHRIFT

(11) **EP 1 663 088 B1**
(45) Veröffentlichungstag und Bekanntmachung des Hinweises auf die Patenterteilung: **20.07.2011**
(21) Anmeldenummer: 04797937.2
(22) Anmeldetag: 16.11.2004
(51) Int. Cl.: A61F 9/009, A61F 9/01

(54) **ADAPTER ZUM MECHANISCHEN KOPPELN EINER LASERBEARBEITUNGSVORRICHTUNG MIT EINEM OBJEKT**
ADAPTER FOR MECHANICALLY COUPLING A LASER PROCESSING DEVICE TO AN OBJECT
ADAPTATEUR DESTINE AU COUPLAGE MECANIQUE D'UN DISPOSITIF D'USINAGE AU LASER ET D'UN OBJET

(30) Priorität: 19.11.2003 DE 10354025
(43) Veröffentlichungstag der Anmeldung: 07.06.2006
(60) Teilanmeldung: 10012107.8
(73) Patentinhaber: Carl Zeiss Meditec AG, 07745 Jena (DE)
(72) Erfinder: MÜHLHOFF, Dirk, 07751 Kunitz (DE); EBERT, Elke, 07743 Jena (DE); FESTAG, Karsten, 07749 Jena (DE); WOLF, Uwe, 99441 Magdala (DE)
(74) Vertreter: Geyer, Fehners & Partner
(86) Internationale Anmeldenummer: PCT/EP2004/012998
(87) Internationale Veröffentlichungsnummer: WO 2005/048895

(56) Entgegenhaltungen:
- EP-A- 0 336 065
- WO-A1-03/002008
- DE-A1- 19 831 674
- US-A- 4 718 418
- US-A1- 2001 021 844

## Beschreibung

Die Erfindung bezieht sich auf eine Kombination einer Laserbearbeitungsvorrichtung und einen Adapter zum mechanischen Koppeln der Laserbearbeitungsvorrichtung mit einem Objekt, wobei der Adapter einen zentralen Bereich, welcher in den auf einer optischen Achse zum Objekt verlaufenden Strahlengang der Laserbearbeitungsvorrichtung schaltbar ist, und einen außerhalb des zentralen Bereichs liegenden Randbereich aufweist und einerseits an der Laserbearbeitungsvorrichtung befestigbar und andererseits mit seinem zentralen Bereich zur Anlage am Objekt ausgebildet ist.

Bei der Materialbearbeitung wird oft eine Laserbearbeitungsvorrichtung zum Abrastern der zu bearbeitenden Gebiete des Objektes mit einem Laserstrahl eingesetzt. Die Genauigkeit der Positionierung des Laserstrahls bestimmt dabei in der Regel die bei der Bearbeitung erzielte Präzision. Wird der Laserstrahl in ein Bearbeitungsvolumen fokussiert, bedarf es einer exakten dreidimensionalen Positionierung. Für eine hochgenaue Bearbeitung ist es deshalb in der Regel unerläßlich, das Objekt in exakt definierter Lage zur Laserbearbeitungsvorrichtung zu halten. Für solche Anwendungen dient der eingangs genannte Adapter, da mit ihm das zu bearbeitende Objekt fixiert werden kann, wodurch definierte Verhältnisse bis zum Bearbeitungsvolumen erreichbar sind. Der zentrale Bereich des Adapters wird damit Teil des Strahlenganges.

Dies ist insbesondere bei der Mikrobearbeitung von Materialien notwendig, die nur eine geringe lineare optische Absorption im Spektralbereich der bearbeitenden Laserstrahlung aufweisen. Bei solchen Materialien werden üblicherweise nicht-lineare Wechselwirkungen zwischen Laserstrahlung und Material ausgenutzt, meist in Form eines optischen Durchbruches, der im Fokus des Laserstrahls erzeugt wird. Da die bearbeitende Wirkung dann nur im Laserstrahlfokus stattfindet, ist es unerläßlich, den Fokuspunkt exakt dreidimensional auszurichten. Zusätzlich zu einer zweidimensionalen Ablenkung des Laserstrahls ist somit eine exakte Tiefeneinstellung der Fokuslage im Strahlengang erforderlich. Der eingangs genannte Adapter dient dazu, konstante und auch mit einer gewissen Genauigkeit bekannte optische Verhältnisse im Strahlengang zum Objekt sicherzustellen, indem sein zentraler Bereich Teil des Strahlenganges ist und er Objekt und Laserbearbeitungsvorrichtung koppelt.

Eine typische Anwendung für einen solchen Adapter ist das als Femtosekunden-LASIK bekannte augenoptische Operationsverfahren, bei dem die als Therapiegerät ausgebildete Laserbearbeitungsvorrichtung einen Laserstrahl auf einen Fokuspunkt in der Größenordnung eines Mikrometers in die Hornhaut fokussiert. Im Fokus entsteht dann ein Plasma, das eine lokale Trennung des Hornhautgewebes bewirkt. Durch geeignete Aneinanderreihung der auf diese Weise erzeugten lokalen Trennungszonen werden mikroskopische Schnitte realisiert und ein bestimmtes Hornhautteilvolumen isoliert. Durch Entnahme des Teilvolumens wird dann eine gewünschte Brechungsänderung der Hornhaut erreicht, so daß eine Fehlsichtigkeitskorrektur möglich ist.

Für das LASIK-Verfahren ist aus der US 6.373.571 eine mit Referenzmarken versehene Kontaktlinse bekannt. Diese Kontaktlinse wird mittels einer separaten Meßvorrichtung einjustiert, wodurch ein relativ aufwendiger Aufbau bedingt ist. Ein Beispiel für einen Adapter der genannten Art ist in der EP 1 159 986 A2 beschrieben. Er ähnelt der Kontaktlinse der US 6.373.571, weist aber zusätzlich einen Rand in Form einer Halterung mit Strichmarken auf, die dem Chirurgen eine visuelle Ausrichtung ermöglichen.

Da der Adapter üblicherweise Kontakt mit dem zu bearbeitenden Objekt hat, ist es meist erforderlich, für jedes Objekt einen eigenen neuen Adapter einzusetzen. Dies gilt besonders unter dem Gesichtspunkt der Sterilität bei augenchirurgischen Verfahren. Daraus folgt, daß der Adapter jedesmal vor einer Bearbeitung bzw. vor einem chirurgischen Eingriff an der Laserbearbeitungsvorrichtung, die dann als Therapiegerät ausgebildet ist, befestigt werden muß. Zur Befestigung ist es aus der WO 03/002008 A1 bekannt, das dort als Applanierungsglas ausgebildete Kontaktglas in einer zangenartigen Einrichtung zu halten, die an der Laserbearbeitungsvorrichtung verriegelt ist. Die Verriegelung erfolgt über einen in einer Schiene geführten Kragen. Der Adapter wird quer zur optischen Achse formschlüssig eingeschoben. Das Kontaktglas wird zuvor in einen elastischen Ring eingelegt, der durch Unterdruck am Auge befestigt wird. Der Ring weist den genannten Kragen auf und wird darüber dann von der zangenartigen Einrichtung mit einem Ende eines Konus verriegelt, der mit seinem anderen Ende an der Laserbearbeitungsvorrichtung fixiert ist. Das Ende des Konus ist somit am elastischen Ring befestigt, welcher wiederum am Auge angesaugt ist, wodurch das Ende des Konus das Kontaktglas auf das Auge drückt und damit die Hornhaut flachpreßt. Als Alternative offenbart diese Druckschrift auch eine Verbindung von elastischem Ring und Konusende mittels Unterdruck oder Magnetkraft und ohne die zangenartige Einrichtung.

In der DE 198 31 674 A1 ist die Verwendung eines mechanischen Kopplungsmechanismus beschrieben, bei dem ein in schrägem Winkel an einer Fassung eines Kontaktglases befestigter Metallstab mittels eines Magneten oder Elektromagneten in einer Hülse gehalten wird. Die Hülse sitzt ihrerseits an einem mechanischen Justiermechanismus, so daß die Stellung des Kontaktglases justiert werden kann.

Die Lösungen im Stand der Technik für das einen Adapter realisierende Kontaktglas benötigen zum einen durchwegs eine große Anzahl von komplex herzustellenden Bauteilen, zum anderen führen die im Stand der Technik verwendeten Befestigungen zu relativ groß bauenden Einheiten. Dies macht Kontaktgläsern des Standes der Technik für Vorrichtungen, die am Patienten ohne Vollanästhesie, sondern mit nur örtlicher Betäubung eingesetzt werden sollen, nachteilig. Nachteilig ist auch die große Menge der zur Herstellung dieses Einwegartikels notwendigen Materials, insbesondere im Hinblick auf Wirtschaftlichkeit und Ökologie.

Der Erfindung liegt deshalb die Aufgabe zugrunde, einen Adapter der eingangs genannten Art baulich hinsichtlich der Befestigung an der Laserbearbeitungsvorrichtung zu vereinfachen, so daß insbesondere eine Anwendung auch ohne Vollnarkose möglich ist. Insbesondere soll eine Anwendung möglich sein, bei der der Adapter während bereits vollständig an der Laserbearbeitungsvorrichtung befestigt unter Lokalanästhesie am Patienten eingesetzt wird.

Diese Aufgabe wird erfindungsgemäß mit einer Kombination aus einer Laserbearbeitungsvorrichtung und einem Adapter gelöst, die die Merkmale des Anspruchs 1 aufweist. Am Randbereich des Adapters sind Befestigungsmittel vorgesehen, mit denen der Adapter lösbar an einer Aufnahme der Laserbearbeitungsvorrichtung so befestigbar ist, daß bei Aufbringen einer bestimmten Zugkraft entlang der optischen Achse die Befestigungsmittel von der Aufnahme lösbar sind.

Der Adapter ist durch eine axiale Bewegung (bezogen auf die optische Achse) an einer Aufnahme der Laserbearbeitungsvorrichtung ansetz- und lösbar und mit einer Unterdruckbefestigung befestigbar.

Die erfindungsgemäße Befestigung des Adapters an einer Aufnahme der Laserbearbeitungsvorrichtung erlaubt es nun, durch eine axiale Bewegung (bezogen auf die optische Achse der Laserbearbeitungsvorrichtung) den Adapter an der Laserbearbeitungsvorrichtung anzusetzen und auch wieder zu lösen. Dieses Vorgehen beruht auf der erstmals von den Erfindern gemachten und im Stand der Technik nicht angesprochenen Erkenntnis, daß es bei der Anwendung eines Adapters für ein augenchirurgisches Verfahren ohne Vollnarkose mitunter zu panikartigen Versuchen des Patienten kommen kann, seinen Kopf von der als Therapiegerät ausgebildeten Laserbearbeitungsvorrichtung wegzubewegen. Der erfindungsgemäße Adapter hat die vorteilhaften Eigenschaften eines "Panikverschlusses", der bei Wegziehen des Kopfes vom Therapiegerät aufgeht.

Der Adapter läßt sich dabei überraschend einfach dadurch realisieren, daß die Befestigungsmittel am Randbereich des Adapters vorgesehen werden, der beim Einsatz des Adapters nicht im Strahlengang der Laserbearbeitungsvorrichtung liegt.

Der erfindungsgemäß vorgesehene Adapter erlaubt eine einfach herzustellende Verbindung mit der Laserbearbeitungsvorrichtung, aufwendige Einsetz- und Justiervorgänge, wie sie im Stand der Technik unerläßlich sind, sind nicht nötig. Insbesondere erfolgt die Befestigung nicht zwingend in einer bestimmten Winkelausrichtung des Adapters, wie es beispielsweise bei der magnetisch arbeitenden Hülse der DE 198 31 674 A1 unerläßlich ist.

Der erfindungsgemäß vorgesehene Adapter ist darüber hinaus schnell und einfach wieder von der Laserbearbeitungsvorrichtung lösbar und öffnet insbesondere im Panikfall bei Überschreiten einer bestimmten Zugkraft entlang der optischen Achse. Schließlich erreicht der erfindungsgemäß vorgesehene Adapter den gewünschten platzsparenden Aufbau.

Die kompakte Bauweise, die für den erfindungsgemäß vorgesehenen Adapter möglich ist, erweist sich überraschenderweise auch aus psychologischen Gründen als Vorteil bei der Verwendung in augenchirurgischen Verfahren. Aufgrund des relativ kleinen, auf das Auge des Patienten aufzusetzenden Adapters empfinden Patienten den Eingriff nunmehr als deutlich weniger gefährdend oder bedrohend, wodurch das Auftreten von Paniksituationen, in denen die Panikverschlußeigenschaft des Adapters zum Tragen käme, deutlich vermindert ist.

Die Ausbildung der am Randbereich des Adapters ansetzenden Befestigungsmittel kann auf vielfältige Art und Weise im Rahmen der Erfindung erfolgen.

Eine Flanschfläche zur Vakuumbefestigung ist vorgesehen. Dann kann der Adapter beispielsweise in eine Bohrung eingelegt werden, die einen Anschlag in Form einer stufenartigen Verjüngung aufweist. Durch Anlegen eines Unterdruckes auf der mikroskopvorrichtungsseitig gelegenen Seite des Adapters, wird der Adapter in die Bohrung auf den Anschlag gezogen und dort gehalten. Mit diesem Konzept ist zugleich eine gewisse Zentrierung des Adapters erreichbar Es ist deshalb bevorzugt, daß die Befestigungsmittel eine am Adapter vorgesehene Flanschfläche zur Vakuumbefestigung aufweisen. Dies ist ein Beispiel für eine Ausgestaltung, bei der die Befestigungsmittel den zentralen Bereich des Adapters zur optischen Achse der Laserbearbeitungsvorrichtung ausrichten. Dabei ist es besonders bevorzugt, die Flanschfläche so auszulegen, daß sie eine geringe Leckrate aufweist. Dadurch ist es möglich, die Flächen großzügig zu tolerieren und kostengünstig herzustellen. Die Vakuumpumpe ist in diesem Fall so dimensioniert, daß sie trotz bzw. bei gegebener Leckrate einen ausreichenden Unterdruck aufrecht erhält.

Möchte man die zentrierende Wirkung der Befestigung verstärken, kann der Randbereich des Adapters kegelstumpfförmig ausgebildet werden. Die Bohrung ist dann ebenfalls kegelförmig auszulegen, so daß der kegelstumpfförmige Adapter vom Unterdruck in die sich verjüngende Bohrung gezogen wird.

In einer alternativen Ausgestaltung dieses selbstzentrierenden Befestigungsmechanismus kann sich der Randbereich des Adapters kegelförmig zur Laserbearbeitungsvorrichtung hin aufweiten. Weist die Laserbearbeitungsvorrichtung eine dazu passende kegelstumpfförmige Aufnahme auf, kann auch dann der Adapter einfach mittels Unterdruck zentrierend an der Laserbearbeitungsvorrichtung befestigt werden.

Zur Verbesserung der optischen Eigenschaften des Adapters ist es ggf. sinnvoll, flüssige Kontaktmittel an dessen optischen Grenzflächen einzusetzen. Dieses in der Mikroskopie bekannte Prinzip der Immersionsoptiken verbessert die Haftungs- und optischen Eigenschaften des Adapters.

Das Anwendungsgebiet für den erfindungsgemäß vorgesehenen Adapter, bei dem dessen Vorteile besonders zur Geltung kommen ist, wie bereits erwähnt, die Augenchirurgie, weshalb der zentrale Bereich des Adapters als Kontaktglas zum Auflegen auf das Objekt, insbesondere auf die Augenhornhaut, ausgebildet ist. Für ein solches Kontaktglas kann man zweckmäßigerweise den Randbereich als Fassung ausgestalten.

Je nach Fertigungsmethode und verwendeten Materialien kann der zentrale Bereich und der Randbereich einstückig gebildet sein. Bei der Verwendung eines Kunststoffmaterials kommt dann beispielsweise ein Spritzgußverfahren für die Herstellung in Frage.

Um die gewünschte feste mechanische Kopplung zwischen Objekt und Laserbearbeitungsvorrichtung sicherzustellen, ist es ratsam, den Adapter nicht nur am Objekt anzulegen, sondern dort auch zu befestigen. Um den zentralen Bereich, der im Strahlengang der Laserbearbeitungsvorrichtung zu liegen kommt, in seiner optischen Funktion möglichst wenig zu beeinträchtigen, bietet es sich dazu an, nötige Fixierungsmittel zur Befestigung am Objekt am Randbereich vorzusehen. Die Fixierungsmittel, hier kommt beispielsweise im Falle augenchirurgischer Verfahren eine Saugkanalstruktur für Unterdruckbefestigung in Frage, bewirken dann, daß der zentrale Bereich im Kontakt mit dem Objekt liegt.

Die Erfindung wird nachfolgend unter Bezugnahme auf die Zeichnungen näher erläutert. In den Zeichnungen zeigt:
- Fig. 1: eine schematische Darstellung einer Laserbearbeitungsvorrichtung für ein augenchirurgisches Verfahren,
- Fig. 2: eine schematische Darstellung der Augenhornhaut eines Patienten,
- Fig. 3: eine Schnittdarstellung durch einen Adapter, der durch Unterdruck befestigt wird,
- Fig. 4: eine Weiterbildung des Adapters der Fig. 3.

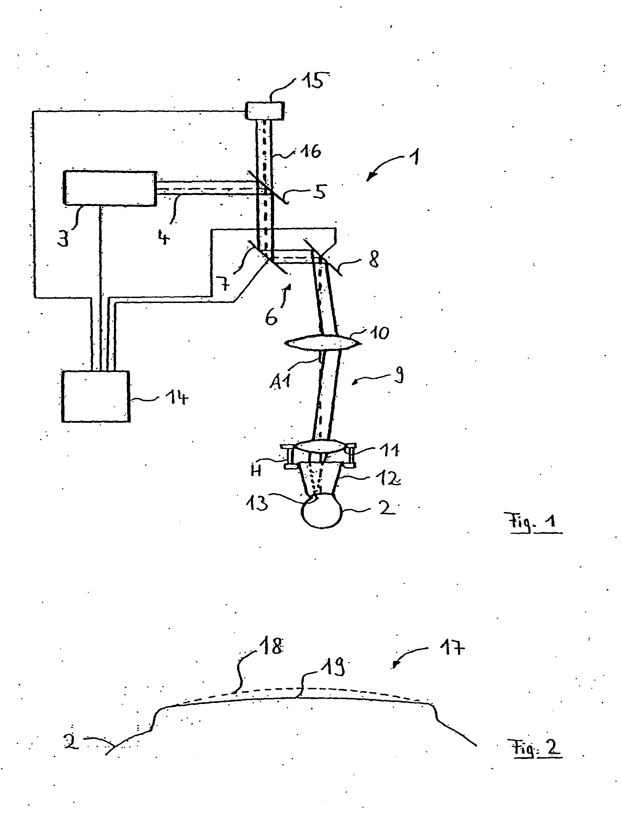

Fig. 1 zeigt ein Behandlungsgerät für ein augenchirurgisches Verfahren ähnlich dem in der EP 1159986 A1 bzw. der US 5549632 beschriebenen. Das Behandlungsgerät 1 der Figur 1 dient dazu, an einem Auge 2 eines Patienten eine Fehlsichtigkeitskorrektur gemäß dem bekannten LASIK-Verfahren auszuführen. Dazu weist das Behandlungsgerät 1 einen Laser 3 auf, der gepulste Laser-Strahlung abgibt. Die Pulsdauer liegt dabei z.B. im Femtosekundenbereich, und die Laserstrahlung wirkt mittels nichtlinearer optischer Effekte in der Hornhaut auf die eingangs beschriebene Art und Weise. Der vom Laser 3 entlang einer optischen Achse A1 abgegebene Behandlungsstrahl 4 fällt dabei auf einen Strahlteiler 5, der den Behandlungsstrahl 4 auf eine Scaneinrichtung 6 leitet. Die Scaneinrichtung 6 weist zwei Scanspiegel 7 und 8 auf, die um zueinander orthogonale Achsen drehbar sind, so daß die Scaneinrichtung 6 den Behandlungsstrahl 4 zweidimensional ablenkt. Eine verstellbare Projektionsoptik 9 fokussiert den Behandlungsstrahl 4 auf bzw. in das Auge 2. Die Projektionsoptik 9 weist dabei zwei Linsen 10 und 11 auf. Das Behandlungsgerät 1 stellt eine Laserbearbeitungsvorrichtung dar.

Der Linse 11 ist ein Adapter 12 nachgeordnet, der über eine Halterung H fest mit der Linse 11 und damit dem Strahlengang des Behandlungsgerätes 1 verbunden ist. Der noch näher zu beschreibende Adapter 12 liegt an der Hornhaut des Auges 2 an. Die optische Kombination aus Behandlungsgerät 1 mit daran befestigtem Adapter 12 bewirkt, daß der Behandlungsstrahl 4 in einem in der Hornhaut des Auges 2 gelegenen Fokus 13 gebündelt wird.

Die Scaneinrichtung 6 wird ebenso wie der Laser 3 und die Projektionsoptik 9 über (nicht näher bezeichnete) Steuerleitungen von einem Steuergerät 14 angesteuert. Das Steuergerät 14 bestimmt dabei die Lage des Fokus 13 sowohl quer zur optischen Achse A1 (durch die Scanspiegel 7 und 8) sowie in Richtung der optischen Achse A1 (durch die Projektionsoptik 9) vor.

Das Steuergerät 14 liest weiter einen Detektor 15 aus, der von der Hornhaut rückgestreute Strahlung, die den Strahlteiler 5 als Rückstrahlung 16 passiert, ausliest. Mittels des Detektors 15 kann der Betrieb des Lasers 3 sehr exakt gesteuert werden.

Der Adapter 12 sorgt dafür, daß die Hornhaut des Auges 2 eine gewünschte Soll-Form erhält. Das Auge 2 befindet sich aufgrund der Anlage der Hornhaut 17 am Adapter 12 in vorbestimmter Lage zum Adapter 12 und damit zum damit verbundenen Behandlungsgerät 1.

Dies ist schematisch in Fig. 2 dargestellt, die einen Schnitt durch die Augenhornhaut 17 zeigt. Um eine exakte Positionierung des Fokus 13 in der Augenhornhaut 17 zu erreichen, muß die Krümmung der Augenhornhaut 17 berücksichtigt werden. Die Augenhornhaut 17 weist eine Ist-Form 18 auf, die von Patient zu Patient unterschiedlich ist. Der Adapter 12 liegt nun an der Augenhornhaut 17 derart an, daß er diese in eine gewünschte Soll-Form 19 verformt. Der genaue Verlauf der Soll-Form 19 hängt dabei von der Krümmung der dem Auge 2 zugewandten Fläche des Adapters 12 ab. Durch den Adapter 12 sind bekannte geometrische und optische Verhältnisse für das Einbringen und Fokussieren des Behandlungsstrahls 4 in die Hornhaut 17 gegeben. Da die Hornhaut 17 am Adapter 12 anliegt und dieser wiederum über die Halterung H gegenüber dem Strahlengang des Behandlungsgerätes 1 ortsfest ist, kann der Fokus 13 durch Ansteuerung der Scaneinrichtung 6 sowie der verstellbaren Projektionsoptik 9 dreidimensional exakt in der Hornhaut 17 positioniert werden.

Der Adapter 12 ist zweiteilig aufgebaut und besteht aus einem Kontaktglas 22, das in eine Fassung 23 geklebt ist. Durch Ansaugen der Fassung 23 auf die Augenhornhaut 17 wird das in die Fassung 23 geklebte Kontaktglas 22 mit seiner Unterseite 29 auf die Augenhornhaut 17 gepreßt, so daß die zuvor bereits erläuterte gewünschte Soll-Form 19 gewährleistet ist.

Anstelle einer zweiteiligen Ausführung kann der Adapter 12 auch einstückig ausgebildet werden. Fassung 23 und Kontaktglas 22 sind dann aus einem durchgehenden Teil hergestellt, beispielsweise durch Spritzgußverfahren oder durch ein zerspanendes Verfahren aus einem einzigen Rohteil. Die hier beschriebenen Varianten des Adapters 12 können grundsätzlich mehrteilig oder auch einteilig realisiert werden, insbesondere was Kontaktglas 22 und Fassung 23 angeht.

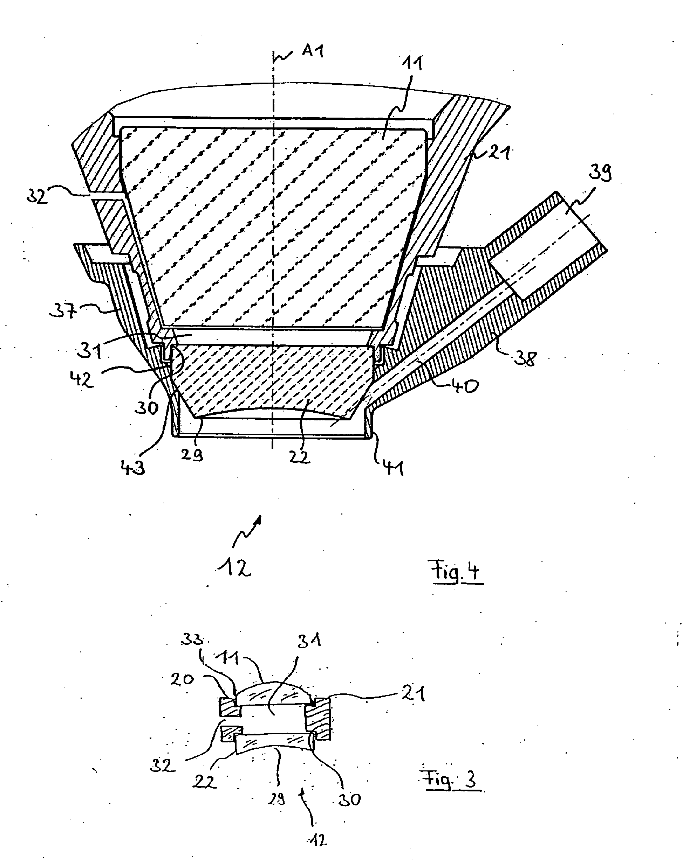

Fig. 3 zeigt eine Ausführungsform des Adapters 12. Hier wird der als fassungsloses Kontaktglas 22 gestaltete Adapter 12 in eine Aufnahme 21 eingeschoben, die an ihrem unteren Ende, d.h. der der Linse 11 abgewandten Seite, eine am Innenumfang umlaufende Stufe hat. Die Aufnahme 21 ist bei dieser Ausführungsform Teil der Linsenfassung 20, die sich röhrenförmig unterhalb der Linse 11 fortsetzt.

Dadurch ist ein Anschlag gebildet, bis zu dem das Kontaktglas 22 in den Adapter 12 eingeschoben werden kann. Das Kontaktglas 22 kommt so mit einem ringförmigen Randbereich an der in der Aufnahme 21 gebildeten Anschlagfläche 30 zu liegen. Kontaktglas 22, Aufnahme 21 sowie Linse 11 bilden einen Hohlraum 31, der über einen seitlichen Unterdruckanschluß 32 evakuiert werden kann. Zur Verbesserung der Dichtigkeit bzw. zur Minderung der erforderlichen Saugleistung ist die Linse 11 in ihre Fassung 20 zusätzlich mittels einer Dichtung, z.B. durch einen Dichtring, oder mittels einer Klebung 33 dichtend eingesetzt.

Das Kontaktglas 22 stützt sich somit durch Unterdruck gehalten mit seinem ringförmigen Randbereich an der Anschlagfläche 30 ab. Durch die Bohrung in der Aufnahme 21 ist dabei zugleich eine Zentrierung des Kontaktglases erreicht. Möchte man diese Zentrierung noch steigern, kann der Randbereich des Kontaktglases 22 kegelstumpfförmig ausgebildet werden und die Bohrung in der Aufnahme 20 erhält eine entsprechende sich verjüngende Kegelform.

Um einen besonders guten Unterdruckaufbau zu erreichen, kann man die Flanschfläche 34 mit einem flexiblen Material, wie z.B. Silikon, versehen, da ein solches Material zu einem besonders guten Luftabschluß und damit zu einer guten Haltekraft führt.
Fig. 4 zeigt einen Adapter 12, der das Befestigungsprinzip des Adapters der Fig. 3 aufgreift. Gleiche Elemente sind mit desselben Bezugszeichen versehen, so daß diesbezüglich auch auf die Beschreibung zu Fig. 3, verwiesen wird. In Abweichung zur Bauweise der Fig. 3 ist das Kontaktglas 22 allerdings mit einem als Fassung dienenden Kontaktglashalter 37 versehen.

Das Kontaktglas 22 sitzt im Kontaktglashalter 37. Es weist einen sich zum Auge hin verjüngenden konischen Abschnitt auf, der an einer konischen Ringfläche 43 des Kontaktglashalters 37 anliegt. Zur Befestigung des Kontaktglases 22 im Kontaktglashalter 37 kann an dieser Stelle eine Verklebung erfolgen. Alternativ oder zusätzlich ist eine Verklebung im Bereich des zylindrischen Kontaktglasabschnittes möglich. Dazu weist zweckmäßigerweise der Kontaktglashalter 37 in der Bauweise der Fig. 4 eine geeignete kegelförmige Abflachung auf, in die nach Einsetzen des Kontaktglases 22 in den Kontaktglashalter 27 Klebstoff eingebracht wird, so daß der Kontaktglashalter 37 an Kontaktglas 22 befestigt ist.

Der Kontaktglashalter 37 wirkt an der Befestigung des Kontaktglases 22 nicht mit, das wie bei der Bauweise der Fig. 3 auch mit einem ringförmigen Randbereich der oberen, d.h. der Laserbearbeitungsvorrichtung zugewandten Fläche mittels Unterdruck an die Anschlagfläche 30 gezogen wird. Der Unterdruck im Hohlraum 31 wird wiederum über einen Unterdruckanschluß 32 aufgebracht, der als Kanal durch die Aufnahme 21 des Behandlungsgerätes 1 ausgebildet ist. Der Kanal kann dabei in der die im Ausführungsbeispiel der Fig. 4 trichterförmig ausgebildete Aufnahme 21 ausgebildet werden. Auch ist es möglich, den Kanal durch eine entsprechende Nut in der Aufnahme 21 zu gestalten, so daß er durch die trichterförmige Aufnahme 21 und die eingesetzte vorderste Linse 11 begrenzt ist. Da die Linse 11 ihrerseits in der Aufnahme 21 dichtend befestigt, beispielsweise verklebt ist, führt ein Anlegen von Unterdruck am Unterdruckanschluß 32 zu einer Evakuierung des Hohlraumes 31, der das Kontaktglas 22 auf die Anschlagfläche 30 zieht. Ein Lösen dieser Unterdruckbefestigung gelingt mit einer Kraft, welche die von der Unterdruckbefestigung ausgeübte Saugkraft überwindet. Durch Einstellen der Saugleistung am Unterdruckanschluß 32 kann man die Kraft für die Panikverschlußfunktion, die zum Lösen des Kontaktglases 22 von der Anschlagfläche 30 nötig ist, wunschgemäß einstellen.

Die Mittel zur Zentrierung des Kontaktglases 22 sind hier beispielhaft als Passung ausgebildet, die von einem zylinderförmigen Kragen gebildet wird, der parallel zur optischen Achse A1 von der Anschlagfläche vorsteht und in den beim Befestigen das Kontaktglas 22 in axialer Richtung auf die Anschlagfläche 30 hin eingeführt wird.

Zur Befestigung des Kontaktglases 22 am Objekt, d.h. am Auge, weist der Kontaktglashalter 37 einen Stutzen 38 auf, der über einen Luer-Lock-Anschluß 39 sowie einen damit verbundenen Saugkanal 40 verfügt. Der Saugkanal 40 mündet seitlich am Kontaktglas 22 oberhalb eines Ansatzringes 41 des Kontaktglashalters 37, der gegenüber der Unterseite 29 des Kontaktglases 22 in axialer Richtung vorsteht. Dieser Ansatzring tritt beim Aufsetzen des Kontaktglases 22 zuerst in Kontakt mit der Augenhornhaut 17. Durch Applikation von Unterdruck am Luer-Lock-Anschluß 39 wird dann die Einheit aus Kontaktglashalter 37 und Kontaktglas 29 auf das Auge hin gezogen, so daß die Unterseite 29 des Kontaktglases wie bereits erläutert, die gewünschte Soll-Form 19 der Augenhornhaut 17 sicherstellt.

Da die Unterdruckverhältnisse beim Ansaugen des Kontaktglases 22 auf die Augenhornhaut 17 sehr viel stärker variieren können, als die Druckverhältnisse im Hohlraum 31 ist es zu bevorzugen, die Unterdruckbefestigung des Kontaktglases 22 mit Kontaktglashalter 37 am Auge sehr viel stärker auszulegen, als die Unterdruckbefestigung des Kontaktglases 22 an der Anschlagfläche 30. Bei einem panikbedingten Lösen des Auges vom Behandlungsgerät 1 geht dann mit einer sehr genau einstellbaren bzw. eingestellten Kraft die Verbindung zwischen Kontaktglas 22 und Aufnahme 21 auf; das Kontaktglas 22 mit Kontaktglashalter 37 verbleibt selbst am Auge.

## Patentansprüche

1. Kombination aus einer Laserbearbeitungsvorrichtung und einem Adapter zum mechanischen Koppeln eines Objektes (17) mit der Laserbearbeitungsvorrichtung (1), die zur Unterdruckbefestigung des Adapters (12) eine Aufnahme (21) mit einer darin durch eine am Innenumfang der Aufnahme (21) umlaufende Stufe gebildeten Anschlagfläche (30) und eine unterdruckdicht in die Aufnahme (21) oberhalb der Stufe der Laser einrichtung zugewandt eingesetzte Linse (11) bereitstellt, wobei
- der Adapter (12) einen zentralen Bereich (22), der als Kontaktglas (22) zum Auflegen auf das Objekt (17) ausgebildet und in den auf einer optischen Achse (A1) zum Objekt (17) verlaufenden Strahlengang der Laserbearbeitungsvorrichtung (1) schaltbar ist, und einen außerhalb des zentralen Bereichs (22) liegenden Randbereich (23) aufweist,
- der Adapter (12) lösbar an der Aufnahme (21) der Laserbearbeitungsvorrichtung befestigbar und mittels einer im wesentlichen entlang der optischen Achse (A1) verlaufenden Einsetzbewegung in die Aufnahme (21) einsetzbar ist, und
- der Adapter (12) zur Unterdruckbefestigung an der Laserbearbeitungsvorrichtung (1) ausgebildet ist, indem seine den zentralen Bereich (22) umfassende, zur Laserbearbeitungsvorrichtung (1) weisende Seite dazu ausgebildet ist, mit der Aufnahme (21) und der Linse (11) einen zur Unterdruckbefestigung zu evakuierenden Hohlraum (31) zu bilden, und indem der ringförmige Randbereich des Adapters (12) als Befestigungsmittel eine Flanschfläche zum Abstützen des Adapters (12) an der Anschlagfläche (30) der Aufnahme (21) aufweist.

2. Kombination nach Anspruch 1, **dadurch gekennzeichnet, daß** die Befestigungsmittel (27, 34) den zentralen Bereich (22) zur optischen Achse (A1) ausrichten.

3. Kombination nach Anspruch 1, **dadurch gekennzeichnet, daß** der Randbereich eine Fassung (23) des Kontaktglases (22) aufweist.

4. Kombination nach einem der Ansprüche 1 oder 2, **dadurch gekennzeichnet, daß** der Randbereich (23) und der zentrale Bereich (22) einstückig ausgebildet sind.

5. Kombination nach einem der obigen Ansprüche, **dadurch gekennzeichnet, daß** am Randbereich (23) Fixierungsmittel (28, 41, 40) vorgesehen sind, mit denen der zentrale Bereich (22) im Kontakt mit dem Objekt (17) haltbar ist.

6. Kombination nach Anspruch 5, **dadurch gekennzeichnet, daß** die Fixierungsmittel (41, 40) für eine Unterdruckbefestigung ausgebildet sind.

7. Kombination nach einem der obigen Ansprüche, **dadurch gekennzeichnet, daß** die Befestigungsmittel des Adapters (12) bei Aufbringen einer bestimmten Zugkraft entlang der optischen Achse (A1) von der Aufnahme (21) lösbar sind.

## Claims

1. A combination of a laser processing device and an adapter for mechanically coupling an object (17) to the laser processing device, wherein for vacuum fixation of the adapter (12), the laser processing device (1) provides a holding fixture (21) comprising a stop face (30) which is formed by a ring shoulder defined at the inner periphery of the holding fixture (21) and a lens (11) which is inserted vacuum tight into the holding fixture (21) above the shoulder and facing the laser processing device, wherein
- the adapter (12) comprises a central region (22) which is provided as a contact glass (22) to be placed on the object (17) and which can be moved into the beam path of the laser processing device (1) which beam path extends up to the object (12) along an optical axis (A1) and which adapter (12) comprises a peripheral region (23) located outside the central region (22),
- said adapter (12) being fixable to the holding fixture (21) of the laser processing device (1) and being insertable into the holding fixture (21) by means of an insertion movement extending substantially along the optical axis (A1), and
- said adapter (12) being provided for vacuum fixation to the laser processing device (1) by the side of the adapter which comprises the central region (22) and faces to the laser processing device (1) being adapted to combine with the holding fixture (21) and the lens (11) to form a vacuum fixation chamber (31) and by the ring-shaped periphery of the adapter (12) comprising, as fixation means, a flange face for supporting the adapter (12) at the stop face (30) of the holding fixture (21).

2. The combination of claim 1, **characterized in that** the fixation means (27, 34) align the central region (22) to the optical axis (A1).

3. The combination of claim 1, wherein the peripheral region is designed as a frame of the contact glass (22).

4. The combination of claims 1 or 2, **characterized in that** the peripheral region (23) and the central region (22) are formed integrally.

5. The combination of any of the above claims, **characterized in that** fixing means (28, 41, 40) are provided at the peripheral region (23) by which fixing means the central region (22) can be held in contact with the object (17).

6. The combination of claim 5, **characterized in that** the fixing means (28, 41, 40) are provided for vacuum fixation.

7. The combination of any of the above claims, **characterized in that** the fixing means of the adapter (12) can be released from the holding fixture (21) upon application of a determined pulling force along the optical axis (A1).

## Revendications

1. Combinaison formée par un dispositif de traitement par laser et un adaptateur pour le couplage mécanique d'un objet (17) avec le dispositif de traitement par laser (1) qui, pour la fixation par dépression de l'adaptateur (12), met à disposition un logement (21) avec une surface de butée (30), formée dans ce dernier par un épaulement périphérique sur le pourtour intérieur du logement (21), et une lentille (11) insérée, de manière étanche à la dépression, dans le logement (21) au-dessus de l'épaulement en étant orientée vers le dispositif de traitement par laser, dans laquelle combinaison
- l'adaptateur (12) comporte une zone centrale (22), qui est réalisée sous la forme d'un verre de contact (22) destiné à être posé sur l'objet (17) et qui peut être montée dans la trajectoire des rayons du dispositif de traitement par laser (1), laquelle s'étend sur un axe optique (A1) vers l'objet (17), et une zone latérale (23) située en dehors de la zone centrale (22),
- l'adaptateur (12) peut être fixé de manière amovible au niveau du logement (21) du dispositif de traitement par laser et peut être inséré dans le logement (21) au moyen d'un mouvement d'introduction qui s'étend sensiblement le long de l'axe optique (A1), et
- l'adaptateur (12) est conçu pour une fixation par dépression au niveau du dispositif de traitement par laser (1), du fait que son côté comprenant la zone centrale (22) et orienté vers le dispositif de traitement par laser (1), est conçu de manière à former avec le logement (21) et la lentille (11) une cavité (31) à mettre sous vide pour la fixation par dépression et du fait que la zone latérale annulaire de l'adaptateur (12) comporte comme moyen de fixation une collerette pour l'appui de l'adaptateur (12) sur la surface de butée (30) du logement (21).

2. Combinaison selon la revendication 1, **caractérisée en ce que** les moyens de fixation (27, 34) alignent la zone centrale (22) par rapport à l'axe optique (A1).

3. Combinaison selon la revendication 1, **caractérisée en ce que** la zone latérale comporte une monture (23) pour le verre de contact (22).

4. Combinaison selon la revendication 1 ou 2, **caractérisée en ce que** la zone latérale (23) et la zone centrale (22) sont réalisées d'un seul tenant.

5. Combinaison selon l'une quelconque des revendications précédentes, **caractérisée en ce qu'**au niveau de la zone latérale (23) sont prévus des moyens de fixation (28, 41, 40), par lesquels la zone centrale (22) peut être maintenue en contact avec l'objet (17).

6. Combinaison selon la revendication 5, **caractérisée en ce que** les moyens de fixation (41, 40) sont réalisés pour une fixation par dépression.

7. Combinaison selon l'une quelconque des revendications précédentes, **caractérisée en ce que** les moyens de fixation de l'adaptateur (12) peuvent être désolidarisés du logement (21) lors de l'application d'une force de traction déterminée le long de l'axe optique (A1).
